# EUROPÄISCHE PATENTSCHRIFT

(11) **EP 1 714 619 B1**
(45) Veröffentlichungstag und Bekanntmachung des Hinweises auf die Patenterteilung: **31.07.2013**
(21) Anmeldenummer: 06007933.2
(22) Anmeldetag: 15.04.2006
(51) Int. Cl.: A61B 17/17, A61B 17/88

(54) **Vorrichtung zum Einbringen einer Bohrung in Knochen**
Instrument for making a bore in a bone
Appareil pour réaliser un trou dans des os

(30) Priorität: 22.04.2005 DE 102005019727
(43) Veröffentlichungstag der Anmeldung: 25.10.2006
(73) Patentinhaber: Karl Storz GmbH & Co. KG, 78532 Tuttlingen (DE)
(72) Erfinder: Berberich, Sascha, 78532 Tuttlingen (DE); Weiler, Andreas, Dr., 13505 Berlin (DE)
(74) Vertreter: Weller, Wolfgang

(56) Entgegenhaltungen:
- US-A1- 2003 009 173
- US-B1- 6 254 605
- US-B1- 6 254 606

## Beschreibung

Die Erfindung betrifft eine Vorrichtung zum zielgerechten Einbringen einer Bohrung mittig im femoralen Ansatz des hinteren Kreuzbandes in einem Kniegelenk, mit einem Hohlschaft, durch den ein Bohrdraht durchführbar ist, wobei am distalen Endbereich des Hohlschaftes der Vorrichtung ein Zielelement angeordnet ist, das eine Kante aufweist.

Derartige Vorrichtungen, sog. femorale Zielgeräte, für ein freihändiges Einbringen eines Bohrdrahtes in einen Femur, werden von der Anmelderin vertrieben.

US 2003/0009173 A1 Basis für den Oberbegriff des Anspruchs 1 betrifft einen femoralen Führer, der zur Rekonstruktion des vorderen Kreuzbandes dient. Der femorale Führer weist ein sich langerstreckendes zylindrisches Element auf, in dem ein hindurchgehender Kanal zum Aufnehmen eines Bohrdrahtes bzw. eines Bohrwerkzeugs ausgebildet ist. An einem distalen Ende des zylindrischen Elements ist ein Zielelement angeordnet, das als Zunge ausgebildet ist. Beim Rekonstruieren des vorderen Kreuzbands wird das distale Ende des femoralen Führers durch eine bereits vorhandene Bohrung in der Tibia an die Operationsstelle gebracht. Der femorale Führer wird derart positioniert, dass eine ebene Anlagefläche des Zielelements außen an den Knochen, in dem ein Bohrkanal bewerkstelligt wird, angelegt wird. Durch ein axiales Verschieben des Führers wird ein spitzenartig ausgebildeter Stift in das Femur eingetrieben, um die Position des Führers zu stabilisieren. Dann wird in den Kanal in dem zylindrischen Element ein Bohrdraht und später ein Bohrwerkzeug eingeführt, mit dem der Bohrkanal in dem Femur bewerkstelligt wird, in den später ein Sehnenersatz eingeschoben wird.

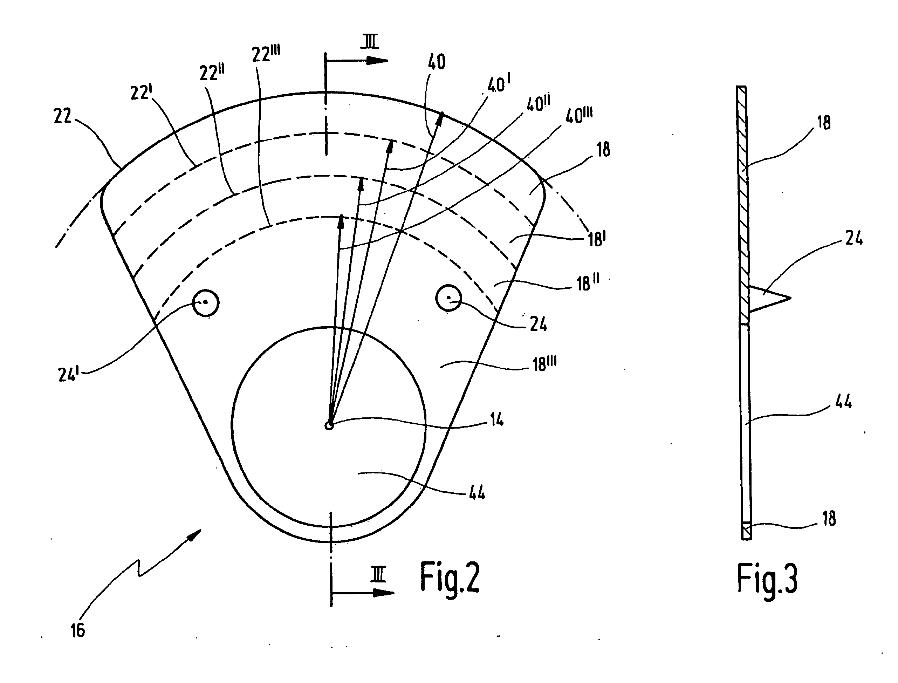

Ein femorales Zielgerät ist in dem Buch "Arthroskopische Chirurgie", PD Dr. med. Michael Strobel, Arthroskopische Chirurgie, Springer Verlag Berlin Heidelberg 1998, Kapitel 2, Seite 513, Abbildungen 2.15-8.a dargestellt.

Dieses Gerät ist in etwa schraubenzieherförmig. Dabei weist das Gerät einen Hohlschaft auf. An seinem proximalen Ende ist der Hohlschaft in einem Handgriff aufgenommen.

Der Hohlschaft verläuft dabei längs entlang der Längsachse des Handgriffs durch den Handgriff hindurch. Durch den Hohlschaft ist ein Bohrdraht durchführbar.

Derartige Vorrichtungen werden auf dem Gebiet der Rekonstruktion des hinteren Kreuzbandes des Kniegelenks eingesetzt.

Im Zentrum des menschlichen Knies liegen zwei sich überkreuzende Bänder, die von der Tibia (Unterschenkelknochen) zum Femur (Oberschenkelknochen) verlaufen, nämlich das vordere und das hintere Kreuzband.

Diese beiden Bänder sorgen zusammen mit den Menisken, den Seitenbändern und Muskeln für die notwendige Stabilität des Kniegelenks und haben dabei eine herausragende Bedeutung.

Bei einem Riss des hinteren Kreuzbandes, muss das verletzte Kniegelenk operativ wieder stabilisiert werden.

Hierzu wird das gerissene hintere Kreuzband durch eine andere Sehne ersetzt. Für diese Kreuzbandrekonstruktion eignen sich die Sehne eines Muskels an der Oberschenkelrückseite, die sog. Semitendinosussehne, oder die Kniescheibensehne, die sog. Patellarsehne.

In beiden Fällen wird die Sehne über Knochenkanäle in Tibia und Femur an der Stelle platziert, an der sich normalerweise das intakte hintere Kreuzband befindet.

Eine anatomische Gegebenheit dabei ist, dass der femorale etwa kreisflächenförmige Ansatz des hinteren Kreuzbandes einen Außenrand aufweist, der teilweise an der Knorpel-Knochen-Grenze des Femurs entlang läuft.

Für die Rekonstruktion des hinteren Kreuzbandes wird das distale Ende des Schaftes des Zielgerätes etwa mittig am femoralen Ansatz des hinteren Kreuzbandes angesetzt. Die Genauigkeit des Ansatzes und auch die Ausrichtung hängt von der Geschicklichkeit des Operateurs ab.

Daraufhin wird durch den Hohlschaft ein Bohrdraht möglichst mittig in diesen femoralen Ansatz gestochen. Anschließend wird dieser Bohrdraht durch den Femur hindurch von intra- nach extraartikulär geschoben.

Der so platzierte Bohrdraht wird mit einem Hohlbohrer überbohrt und dadurch ein Bohrkanal in den Femur eingebracht.

Der Bohrkanal wird so angelegt, dass er zwei Teilabschnitte aufweist. Von intra- nach extraartikulär gesehen ist der Durchmesser des ersten Abschnitts dabei größer als der des zweiten Abschnitts.

Der Kreuzbandersatz wird dann üblicherweise als Doppelschlinge in den ersten Teilabschnitt des Bohrkanals eingeführt. Durch den Bogen dieser Doppelschlinge wird ein Befestigungsfaden gezogen. Dieser Befestigungsfaden verläuft durch den zweiten Teilabschnitt des Bohrkanals. Am medialen äußeren Ende des Bohrkanals wird der Befestigungsfaden mit einem Fixationsknopf fixiert.

Die freien Enden des Kreuzbandersatzes schließlich werden durch einen tibialen Bohrkanal geführt und z.B. mit einem Knochendübel fixiert.

Bei jeder Kreuzbandrekonstruktion ist die Anlage der Bohrkanäle ein kritischer Operationsschritt.

Die Lage und der Verlauf des femoralen Bohrkanals bestimmen, ob das rekonstruierte hintere Kreuzband anatomisch gleich am Femur verankert ist bzw. anatomisch gleich vom Femur vorspringt wie das natürliche hintere Kreuzband.

Dabei ist das Einbringen einer Bohrung als zielgerecht zu bezeichnen, wenn der eingebrachte Bohrkanal genau so lokalisiert ist, dass das Ersatzkreuzband anatomisch gleich vom Femur vorspringt wie das natürliche hintere Kreuzband.

Eine zielgerechte und mittige Durchführung des Bohrdrahtes durch den femoralen Ansatz des hinteren Kreuzbandes ist dafür unerlässlich. Schon kleinere Abweichungen können zu späteren Instabilitäten im Kniegelenk des Patienten führen.

Im praktischen Einsatz wurde nun festgestellt, dass aufgrund anatomischer Gegebenheiten bzw. einem freihändigen Setzen des Bohrdrahtes die Position des Bohrkanals von der natürlichen Lage des femoralen Ansatzes des hinteren Kreuzbandes abweichen kann. Dies führt zu merklichen Instabilitäten im operierten Kniegelenk.

Es ist daher Aufgabe der vorliegenden Erfindung, hier Abhilfe zu schaffen und eine Vorrichtung dahingehend zu gestalten, dass sie ein ziel- und lagegerechtes Einbringen der Bohrung erleichtert.

Erfindungsgemäß wird die Aufgabe dadurch gelöst, dass die Kante zum Anlegen an den Außenrand des femoralen Ansatzes des hinteren Kreuzbandes geeignet und eine Krümmung aufweist, die dem Außenrand des femoralen Ansatzes des hinteren Kreuzbandes angepasst ist.

Diese Maßnahme hat den Vorteil, dass die Kante des Zielelementes eine Orientierungshilfe für das Anlegen an den Außenrand bietet. Der Außenrand ist während der Operation gut sichtbar.

Der Operateur legt die Kante des Zielelements an den Außenrand des femoralen Ansatzes an, und hat dadurch einen anatomisch richtigen Ausgangspunkt für das Setzen und Ausrichten des Bohrdrahtes.

Der radiale Abstand des Mittelpunktes des Hohlschaftes zur Kante des Zielelementes ist bekannt.

Deshalb durchsticht ein eingeschobener Bohrdraht den femoralen Ansatz mittig. Die Kante gibt auch zugleich die Orientierung der Ausrichtung des Hohlschaftes vor.

Das bedeutet, ein damit eingebrachter Bohrkanal nimmt das Ersatzkreuzband so auf, dass es anatomisch gleich vom Femur vorspringt wie das natürliche hintere Kreuzband.

Der distale Endbereich des Hohlschaftes lässt sich also mit Hilfe der Kante anatomisch genau orientiert im Bereich des femoralen Ansatzes des hinteren Kreuzbandes ansetzen. Dies ergibt ein mittiges und zielgerechtes Einstechen eines Bohrdrahtes.

Ferner hat diese Maßnahme den Vorteil, dass eine in ihrer Krümmung dem Außenrand angepasste Kante passend an den Außenrand des hinteren Kreuzbandes anlegbar ist. Ein Anvisieren des Außenrandes ist mit Hilfe der angepassten Kante erleichtert. Die Konturen des Außenrandes und der Kante lassen sich optisch gut miteinander vergleichen und gut aneinander ausrichten.

Somit ist eine Platzierung des Hohlschaftes am femoralen Ansatz des hinteren Kreuzbandes noch genauer an der Anatomie ausgerichtet. Dies erleichtert weiterhin das zielgerechte Einstechen des Bohrdrahtes. Wie bereits ausgeführt, geschieht die Rekonstruktion des hinteren Kreuzbandes dadurch äußerst angepasst an die anatomischen Gegebenheiten.

In einer weiteren Ausgestaltung der Erfindung entspricht der radiale Abstand der Kante vom Mittelpunkt des Hohlschaftes in etwa dem Radius des Außenrandes des femoralen Ansatzes des hinteren Kreuzbandes.

Diese Maßnahme hat den erheblichen Vorteil, dass die Lage des Mittelpunktes zur Kante genau den anatomischen Gegebenheiten des zu ersetzenden Kreuzbandes angepasst ist. Der Bohdraht wird zwangsläufig genau mittig im femoralen Ansatz angesetzt und durch den Hohlschaft geführt.

Der anschließend mittels eines Hohlbohrers in den Femur eingebrachte Bohrkanal hat die gewünschte Ausrichtung. Sein Austrittsquerschnitt liegt in der Ebene in der der femorale Ansatz des natürlichen hinteren Kreuzbandes liegt. In diesen Bohrkanal eingebrachte Sehnen ersetzen daher das gerissene Kreuzband so naturgetreu wie möglich.

In einer weiteren Ausgestaltung der Erfindung ist das Zielelement abnehmbar angeordnet.

Diese Maßnahme hat den Vorteil, dass die Vorrichtung mit ihrem Zielelement den vorgefundenen anatomischen Gegebenheiten anpassbar ist. Die Form und der Zustand anatomischer Gegebenheiten von Kniegelenken variieren von Patient zu Patient durchaus in weiten Bereichen. Die Größe des Femurs, die genaue Lage des femoralen Ansatzes des hinteren Kreuzbandes und der Grad der Schädigung des Kniegelenks sind nur einige Parameter. Ein abnehmbares Zielelement ist durch andere angepasstere Zielelemente austauschbar. Die Abnehmbarkeit kann so ausgestaltet sein, dass das Zielelement abnehmbar am Hohlschaft angeordnet ist. Es ist auch möglich Hohlschaft und Zielelement fest miteinander zu verbinden und diesen Zusammenbau mit unterschiedlichen Handhaben bzw. Griffen zu verwenden.

In einer weiteren Ausgestaltung der Erfindung sind mehrere Zielelemente mit unterschiedlichen radialen Abständen der Kante vom Mittelpunkt des Hohlschaftes vorhanden.

Diese Maßnahme hat den Vorteil, dass die Vorrichtung in einem weiten Spektrum anatomischer Gegebenheiten anwendbar ist. Mit der Ausgestaltung unterschiedlicher Krümmungsradien ist die Vorrichtung daher leichter an unterschiedlich große femorale Ansätze anzupassen, als z.B. für kleine, große, dünne oder kräftige Personen.

In einer weiteren Ausgestaltung ist das Zielelement als scheibenartiger Körper ausgestaltet.

Diese Maßnahme hat den Vorteil, dass das Zielelement stabil ausgeführt ist. Ein solches Zielelement ist besser durch den Operateur anzulegen und erhöht damit die Sicherheit der Operation. Die Stabilität bzw. die Robustheit der Vorrichtung im operativen Einsatz ist damit generell erhöht.

In einer weiteren Ausgestaltung der Erfindung weist der scheibenartige Körper eine kreisförmige Kante auf.

Diese Maßnahme hat den Vorteil, dass der scheibenartige Körper einfach zu fertigen ist. Darüber hinaus erleichtert eine kreisförmige Kante das Anlegen der Kante an einen kreisförmigen femoralen Ansatz des hinteren Kreuzbandes.

In einer weiteren Ausgestaltung der Erfindung erstreckt sich die kreisförmige Kante nur um einen Umfang eines Teil eines Kreises.

Diese Maßnahme hat den Vorteil, dass die Kante einen ausreichend langen Verlauf hat, um genau angelegt zu werden. Ausserdem bleibt dem Operateur während des Eingriffs genügend Sicht auf den femoralen Ansatz des hinteren Kreuzbandes. Die Ausbildung etwa eines Viertelkreises gibt ausreichende Anlagelänge und ausreichende Sichtmöglichkeiten.

In einer weiteren Ausgestaltung der Erfindung weist der scheibenartige Körper eine Öffnung zum Aufsetzen auf den Hohlschaft auf.

Diese Maßnahme hat den Vorteil, dass der scheibenartige Körper einfach vom Hohlschaft abgenommen bzw. ausgewechselt werden kann. Man schiebt den scheibenartigen Körper über das distale Ende des Hohlschaftes. Abschließend lässt sich die Scheibe mit einer Mutter festlegen.

In einer weiteren Ausgestaltung der Erfindung ist die Öffnung unrund.

Diese Maßnahme hat zusätzlich den Vorteil, dass der aufgesetzte scheibenartige Körper gegen ein seitliches Verdrehen sicherbar ist. Ein scheibenartiger Körper mit beispielsweise viereckig ausgestalteter Öffnung lässt sich einfach über einen distalen Endbereich mit einem entsprechenden viereckigen Querschnitt schieben. Anschließend wird der distale Endbereich verschraubt. Greifen nun seitlich ansetzende Kräfte an dem scheibenartigen Körper an, so werden diese vom scheibenartigen Körper auf die Auflageflächen des Hohlschaftes abgeleitet.

In einer weiteren Ausgestaltung der Erfindung ist die Öffnung rund.

Diese Maßnahme hat den Vorteil, dass der aufgesetzte scheibenartige Körper seitlich um die Schaftachse verdrehbar ist. Stellt der Operateur fest, dass ein um die Längsachse des Hohlschaftes radial seitlich verdrehter scheibenartiger Körper der Anatomie im Kniegelenk besser entspricht, lässt sich die Stellung des scheibenartigen Körpers entsprechend vornehmen.

Damit ist jegliche Stellung des scheibenartigen Körpers um den Hohlschaft herum einstellbar, die sowohl den anatomischen Gegebenheiten entspricht als auch dem Operateur eine ergonomische Handhabung der Vorrichtung erlaubt.

In einer weiteren Ausgestaltung der Erfindung springt von dem scheibenartigen Körper nach distal zumindest ein Stützelement vor.

Diese Maßnahme hat den Vorteil, dass sich die Vorrichtung mit einem Stützelement ihres Zielelementes am femoralen Ansatz des hinteren Kreuzbandes abstützen lässt. Dadurch wird ein Weggleiten der Vorrichtung während der Einbringung des Bohrdrahtes verhindert.

In einer weiteren Ausgestaltung der Erfindung springen mehrere stiftartige Stützelemente vor.

Diese Maßnahme hat den Vorteil, dass die Vorrichtung mit mehreren stiftartigen Stützelementen ihres Zielelementes am femoralen Ansatz des hinteren Kreuzbandes einstechen lässt. Dies gibt einen festen Halt bei der Einbringung des Bohrdrahtes, auch wenn anatomische Unebenheiten ein Verwackeln der Vorrichtung provozieren könnten.

In einer weiteren Ausgestaltung der Erfindung ist am proximalen Endbereich des Hohlschaftes ein abgewinkelter Handgriff angeordnet.

Diese Maßnahme hat den Vorteil, dass die Vorrichtung in der Hand des Operateurs gut handhabbar ist. Ein abgewinkelter Handgriff lässt sich durch den Operateur so führen, dass seine Hand während der Operation nicht in einer unnatürlich gewinkelten Stellung gehalten werden muss. Diese Ausführung sichert ein ergonomisches Arbeiten mit der Vorrichtung.

Darüberhinaus sichert ein diametral zum scheibenartigen Körper angebrachter abgewinkelter Handgriff ein leichtes Anvisieren des femoralen Ansatzes des hinteren Kreuzbandes.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Ansicht einer erfindungsgemäßen Vorrichtung zur Vorbereitung eines zielgerechten Einbringens einer Bohrung,
- Fig. 2: eine Ansicht von distal auf verschiedene Ausführungen von Zielelementen der erfindungsgemäßen Vorrichtung, wobei sich die verschiedenen Zielelemente in ihren Krümmungsradien unterscheiden,
- Fig. 3: einen Schnitt längs der Linie III-III der Fig. 2,
- Fig. 4: einen Schnitt längs der Linie V-V der Fig. 5,
- Fig. 5: eine stark vergrößerte abschnittsweise Ansicht eines distalen Endbereichs der Vorrichtung von Fig. 1, ohne den Bohrdraht,
- Fig. 6: eine schematische Skizze eines menschlichen rechten Kniegelenks mit Femur und Tibia sowie vorderem und hinterem Kreuzband,
- Fig. 7: eine der Fig. 6 vergleichbare Darstellung, wobei ein Einsatz der erfindungsgemäßen Vorrichtung gezeigt ist,
- Fig. 8: eine der Fig. 6 vergleichbare Darstellung, wobei die Rekonstruktion des hinteren Kreuzbandes gezeigt ist.

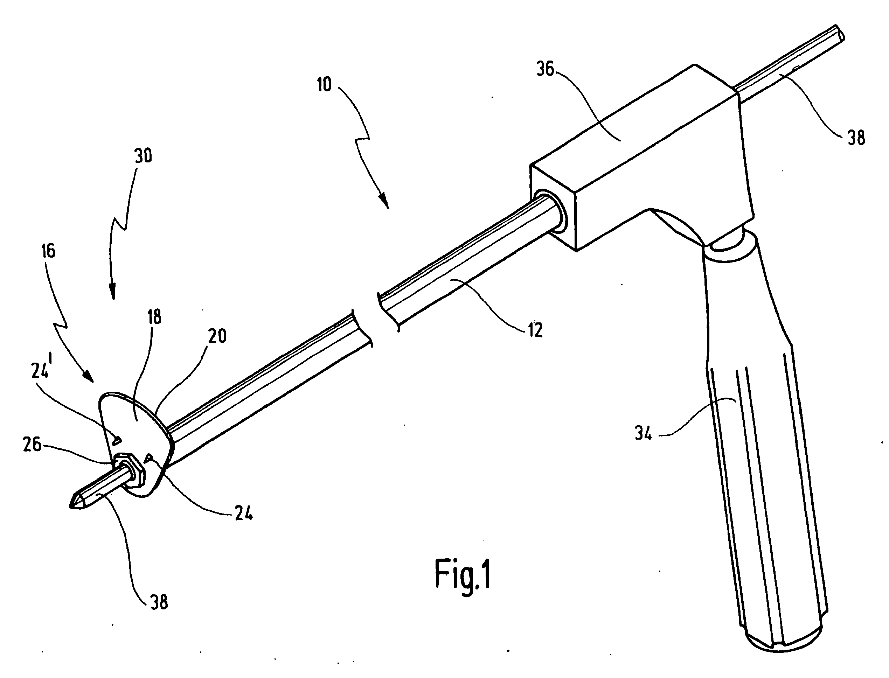

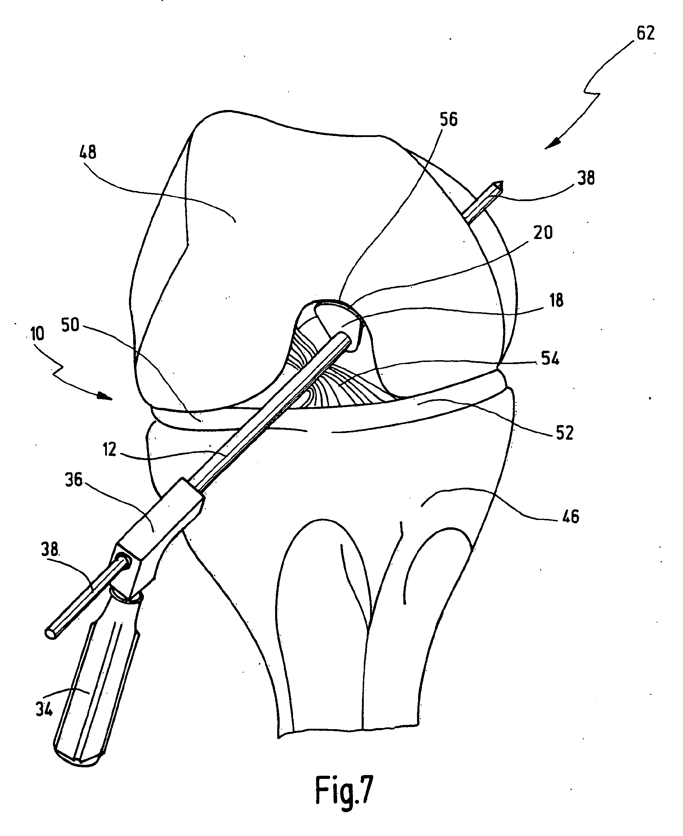

In den Figuren 1 und 7 ist eine Vorrichtung für ein zielgerechtes Einbringen einer Bohrung dargestellt, die in ihrer Gesamtheit mit der Bezugsziffer 10 bezeichnet ist.

Die erfindungsgemäße Vorrichtung 10 weist einen Hohlschaft 12 auf. Der Hohlschaft 12 ist an einem proximalen Endbereich in einem Gehäuse 36 aufgenommen. Der Hohlschaft 12 ist durch dieses Gehäuse 36 hindurchgeführt. An einer proximalen Austrittsstelle schließt der Hohlschaft 12 bündig mit dem Gehäuse 36 ab. Durch diese Austrittsstelle ist ein Bohrdraht 38 durchführbar, wie in Fig. 1 und 7 gezeigt.

Ein Handgriff 34 steht etwa rechtwinklig abgewinkelt zur Längsachse des Hohlschaftes 12 vom Gehäuse 36 ab. Dieser Handgriff 34 ist für ein Ergreifen durch die menschliche Hand vorgesehen.

An einem distalen Endbereich 30 des Hohlschaftes 12 ist ein Zielelement 16 angeordnet. Dieses Zielelement 16 erstreckt sich in etwa diametral zum Handgriff 34.

Dieses Zielelement 16 ist als ein scheibenartiger Körper 18 ausgeformt. Der Körper 18 hat grob die Form einer viertel Scheibe. Der scheibenartige Körper weist eine Kante 20 auf. Die Kante 20 verläuft am scheibenartigen Körper 18 dem Viertelkreis entsprechend umfänglich und kreisförmig. Es ist auch vorgesehen, Körper mit mehr als einem Viertelkreis zur Verfügung zu stellen.

Weiterhin sind an dem scheibenartigen Körper 18, wie in den Figuren 1 bis 5 dargestellt, zwei stiftartige Stützelemente 24 und 24' vorgesehen. Diese stiftartigen Stützelemente 24 und 24' springen von einer Seite des scheibenartigen Körpers nach distal vor.

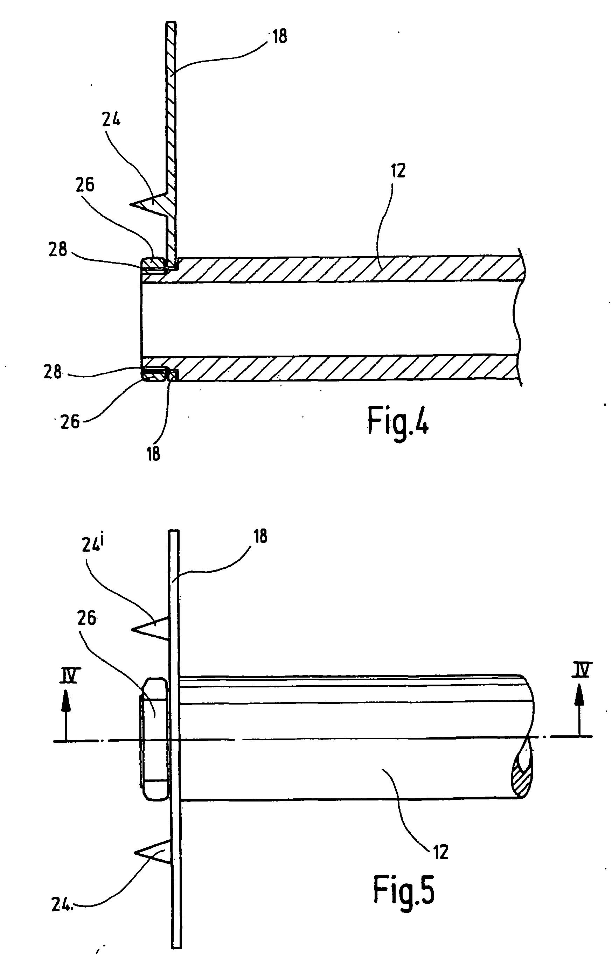

Darüber hinaus ist in dem scheibenartigen Körper 18 eine Öffnung 44 vorhanden. Das Zielelement 16 ist abnehmbar am distalen Endbereich 30 des Hohlschaftes 12 angeordnet. Dazu lässt sich der scheibenartige Körper 18 des Zielelementes 16 mit seiner Öffnung 44 über einen mit einem Außengewinde 28 versehenen Fortsatz schieben. Auf diesem Außengewinde 28 wird anschließend eine Mutter 26 gedreht. Der verschraubte distale Endbereich 30 der erfindungsgemäßen Vorrichtung 10 ist in Fig. 5 in einer vergrößerten abschnittsweisen Ansicht dargestellt.

Verschiedene mögliche Ausführungen des Zielelementes 16 sind in den Figuren 2 und 3 angedeutet.

Dabei handelt es sich jeweils um scheibenartigen Körper 18, 18', 18'' und 18'''. Diese weisen jeweils unterschiedliche radiale Abstände 40, 40', 40" und 40''' auf. Dabei entspringen diese alle einem selben Mittelpunkt 14 des Hohlschaftes 12. Um diesen Mittelpunkt 14 ist in den scheibenartigen Körpern 18 bis 18''' jeweils die Öffnung 44 ausgenommen.

Die unterschiedlichen radialen Abstände 40 bis 40''' sind in Fig. 2 als Pfeile dargestellt. Deren kreisförmiger Verlauf ist in der Fig. 2 gestrichelt eingezeichnet. Mit dieser Abbildung ist auch verdeutlicht, dass die scheibenartigen Körper 18 bis 18''' in ihrer Form alle in etwa Viertelkreisscheiben entsprechen. Dabei weisen sie kreisförmig verlaufende Krümmungen 22 bis 22''' auf. Die Radien entsprechen etwa 7, 8, 9 und 10 mm.

Alle diese scheibenartigen Körper 18 bis 18''' weisen stiftartige Stützelemente 24 bzw. 24' auf.

Fig. 4 zeigt eine vergrößerte abschnittsweise Ansicht des distalen Endbereichs 30. Das Zielelement 16 ist an der Vorrichtung 10 mit der Mutter 26 fixiert. Es ist auch vorgesehen, das Zielelement mit dem Hohlschaft fest zu verbinden z.B. zu verschweißen und jeweils unterschiedliche Größen und/oder Formen des Zielelements als solcher Zusammenbau zur Verfügung zu stellen, die dann mit einem Handgriff lösbar verbunden sind.

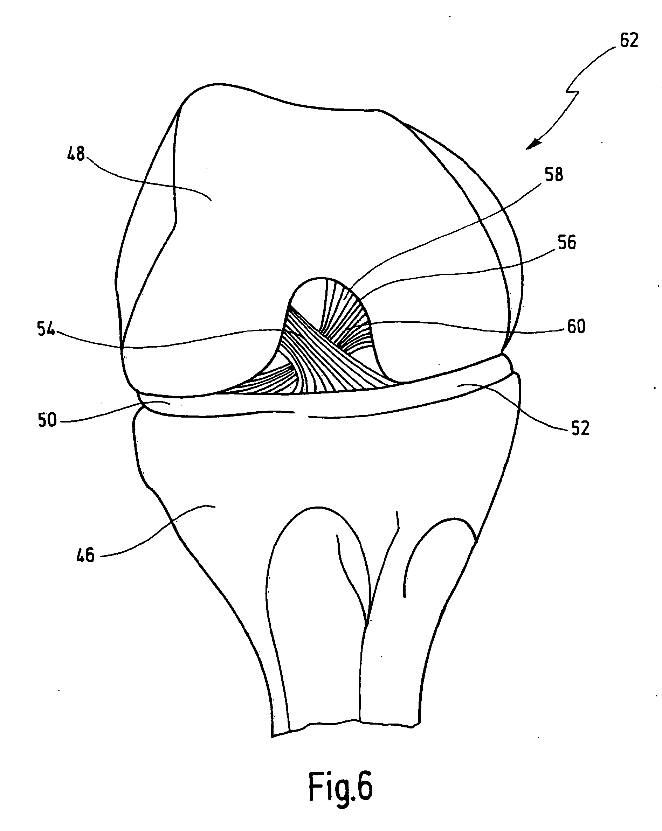

Anhand der Figuren 6 bis 8 soll die Handhabung der Vorrichtung 10 kurz erläutert werden.

In der Fig. 6 ist ein gesundes menschliches Kniegelenk 62 schematisch dargestellt.

Zwischen Tibia 46 und einen Femur 48 befinden sich ein lateraler Meniskus 50 und ein medialer Meniskus 52. Tibia 46 und Femur 48 sind über ein vorderes Kreuzband 54 und ein hinteres Kreuzband 60 verbunden. Das hintere Kreuzband 60 weist einen femoralen Ansatz auf, der von einem etwa kreisförmigen Außenrand 56 umgeben ist.

Bei der Rekonstruktion eines abgerissenen hinteren Kreuzbandes, wie in Fig. 7 dargestellt, wird die Vorrichtung 10 von anterolateral eingebracht und das Zielelement 16 wird mit seiner Kante 20 an den Außenrand 56 des femoralen Ansatzes 58 des hinteren Kreuzbandes angelegt. Durch entsprechende Auswahl der Größe des Zielelements 16, liegt die Längsachse des Hohlschaftes 12 im anatomischen Mittelpunkt des Ansatzes des hinteren Kreuzbandes. Gleichzeitig ist die Orientierung so, dass diese Längsachse etwa senkrecht zur Ansatzfläche steht.

Der Bohrdraht 38 wird zielgerecht und mittig in den femoralen Ansatz 58 hinein und durch den Femur 48 hindurch geschoben.

Die Stützelemente 24 und 24' gewähren dabei einen festen Halt und verhindern ein Abgleiten oder ein Wegdrehen der Vorrichtung 10.

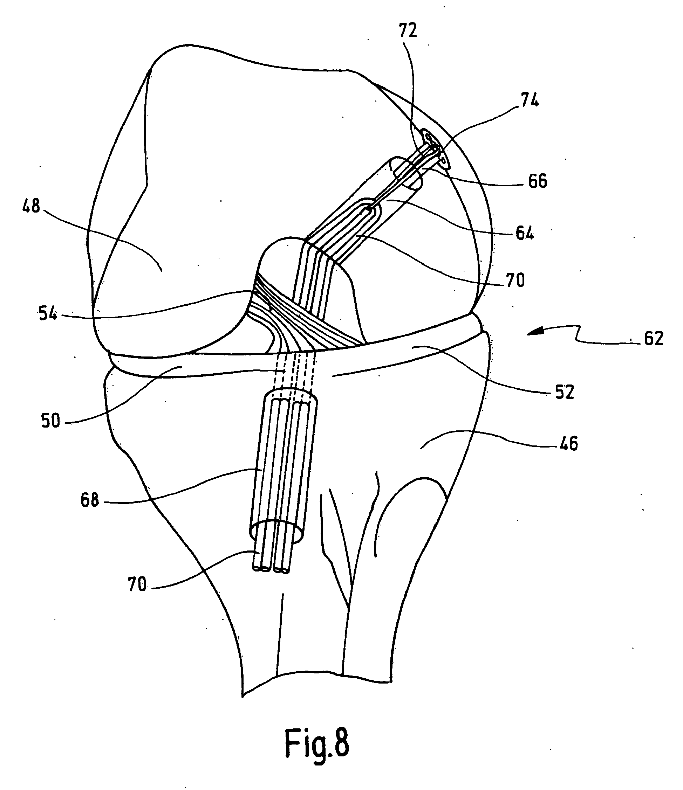

Anschließend wird die Vorrichtung 10 vom Bohrdraht 38 abgezogen und ein Hohlbohrer wird über den Bohrdraht 38 geschoben. Mit Hilfe dieses Hohlbohrers der vom Bohrdraht 38 geführt ist, wird vom femoralen Ansatz 58 ausgehend zielgerecht und mittig gebohrt. Die beiden Teilabschnitte des Bohrkanals 64 bzw. 66 mit ihren unterschiedlichen Durchmessern sind in Fig. 8 dargestellt.

Eine präparierte Sehne wird als ein Kreuzbandersatz 70 in den so vorbereiteten Femur 48 als Doppelschlinge eingebracht. Sie wird von einem Halteband 72 gehalten, das durch einen Fixationsknopf 74 gezogen ist.

In einem tibialen Bohrkanal 68 werden die Enden des Kreuzbandersatzes 70 z.B. über einen Knochendübel oder einen weiteren Fixationsknopf fixiert.

## Patentansprüche

1. Vorrichtung zum zielgerechten Einbringen einer Bohrung mittig im femoralen Ansatz (58) des hinteren Kreuzbandes (60) in einem Kniegelenk (62), mit einem Hohlschaft (12), durch den ein Bohrdraht (38) durchführbar ist, wobei am distalen Endbereich (30) des Hohlschaftes (12) der Vorrichtung ein Zielelement (16) angeordnet ist, das eine Kante (20) aufweist, **dadurch gekennzeichnet, dass** die Kante (20) zum Anlegen an den Außenrand (56) des femoralen Ansatzes (58) des hinteren Kreuzbandes (60) geeignet ist und eine Krümmung (22) aufweist, die dem Außenrand (56) des femoralen Ansatzes (58) des hinteren Kreuzbandes (60) angepasst ist.

2. Vorrichtung nach Anspruch 1, **dadurch gekennzeichnet, dass** der radiale Abstand (40) der Kante (20) vom Mittelpunkt (14) des Hohlschaftes (12) in etwa dem Radius des Außenrandes (56) des femoralen Ansatzes (58) des hinteren Kreuzbandes (60) entspricht.

3. Vorrichtung nach Anspruch 1 oder 2, **dadurch gekennzeichnet, dass** das Zielelement (16) abnehmbar angeordnet ist.

4. Vorrichtung nach Anspruch 2 oder 3, **dadurch gekennzeichnet, dass** mehrere Zielelemente (16), (16'), (16"), (16"') mit unterschiedlichen radialen Abständen (40), (40'), (40"), (40"') der Kante (20) vom Mittelpunkt (14) des Hohlschaftes (12) vorhanden sind.

5. Vorrichtung nach Anspruch 1, 3 oder 4, **dadurch gekennzeichnet, dass** das Zielelement (16) als scheibenartiger Körper (18) ausgestaltet ist.

6. Vorrichtung nach Anspruch 5, **dadurch gekennzeichnet, dass** der scheibenartige Körper (18) eine kreisförmige Kante (32) aufweist.

7. Vorrichtung nach Anspruch 6, **dadurch gekennzeichnet, dass** sich die kreisförmige Kante (32) etwa über einen Umfang eines Teils eines Kreises erstreckt.

8. Vorrichtung nach einem der Ansprüche 5 bis 7, **dadurch gekennzeichnet, dass** der scheibenartige Körper (18) eine Öffnung (44) zum Aufsetzen auf den Hohlschaft (12) aufweist.

9. Vorrichtung nach Anspruch 8, **dadurch gekennzeichnet, dass** die Öffnung (44) unrund ist.

10. Vorrichtung nach Anspruch 8, **dadurch gekennzeichnet, dass** die Öffnung (44) rund ist.

11. Vorrichtung nach einem der Ansprüche 5 bis 10, **dadurch gekennzeichnet, dass** von dem scheibenartigen Körper (18) nach distal zumindest ein Stützelement (24) vorspringt.

12. Vorrichtung nach Anspruch 11, **dadurch gekennzeichnet, dass** mehrere stiftartige Stützelemente (24), (24') vorspringen.

13. Vorrichtung nach einem der Ansprüche 1 bis 12, **dadurch gekennzeichnet, dass** am proximalen Endbereich (42) des Hohlschaftes (12) ein abgewinkelter Handgriff (34) angeordnet ist.

## Claims

1. Device for a correctly targeted formation of a drill hole centrally in the femoral attachment (58) of the posterior cruciate ligament (60) of a knee joint, with a hollow shaft (12) through which a drill wire (38) can be guided, wherein a sighting element (16) having an edge (20) is arranged on the distal end area (30) of the hollow shaft (12) of the device, **characterized in that** the edge (20) is suited to be placed at the outer margin (56) of the femoral attachment (58) of the posterior cruciate ligament (60), and has a curvature (22) which is adapted to the outer margin (56) of the femoral attachment (58) of the posterior cruciate ligament (60).

2. Device of claim 1, **characterized in that** the radial distance (40) of the edge (20) from the center point (14) of the hollow shaft (12) corresponds approximately to the radius of the outer margin (56) of the femoral attachment (58) of the posterior cruciate ligament (60).

3. Device of claims 1 or 2, **characterized in that** the sighting element (16) is arranged in such a way that it can be detached.

4. Device of claims 2 or 3, **characterized in that** several sighting elements (16, 16', 16", 16"') with different radial distances (40, 40', 40", 40"') of the edge (20) from the center point (14) of the hollow shaft (12) are provided.

5. Device of claims 1, 3 or 4, **characterized in that** the sighting element is designed as a disk-like body (18).

6. Device of claim 5, **characterized in that** the disk-like body (18) has a circular edge (32).

7. Device of claim 6, **characterized in that** the circular edge (32) extends for instance about the circumference of a part of a circle.

8. Device of anyone of claims 5 through 7, **characterized in that** the disk-like body (18) has an opening (44) for placing it onto the hollow shaft (12).

9. Device of claim 8, **characterized in that** the opening (44) is not round.

10. Device of claim 8, **characterized in that** the opening (44) is round.

11. Device of anyone of claims 5 through 10, **characterized in that** at least one support element (24) protrudes distally from the disk-like body (18).

12. Device of claim 111, **characterized in that** several pin-like support elements (24, 24') protrude.

13. Device of anyone of claims 1 through 12, **characterized in that** an angled handgrip (34) is arranged at the proximal end area (42) of the hollow shaft (12).

## Revendications

1. Dispositif dévolu au façonnage ciblé d'un perçage au centre de la saillie fémorale (58) du ligament croisé postérieur (60), dans une articulation du genou (62), comprenant une tige creuse (12) pouvant être parcourue par une tringle métallique perforatrice (38), un élément de ciblage (16), muni d'une arête (20), étant situé dans la région extrême distale (30) de ladite tige creuse (12) du dispositif, **caractérisé par le fait que** l'arête (20) se prête à la mise en applique contre le bord extérieur (56) de la saillie fémorale (58) du ligament croisé postérieur (60), et présente une courbure (22) adaptée audit bord extérieur (56) de ladite saillie fémorale (58) dudit ligament croisé postérieur (60).

2. Dispositif selon la revendication 1, **caractérisé par le fait que** la distance radiale (40), comprise entre l'arête (20) et le centre (14) de la tige creuse (12), correspond approximativement au rayon du bord extérieur (56) de la saillie fémorale (58) du ligament croisé postérieur (60).

3. Dispositif selon la revendication 1 ou 2, **caractérisé par le fait que** l'élément de ciblage (16) est implanté de manière amovible.

4. Dispositif selon la revendication 2 ou 3, **caractérisé par** la présence de plusieurs éléments de ciblage (16, 16', 16", 16"') présentant des distances radiales différentes (40, 40', 40", 40"') entre l'arête (20) et le centre (14) de la tige creuse (12).

5. Dispositif selon la revendication 1, 3 ou 4, **caractérisé par le fait que** l'élément de ciblage (16) est configuré en un corps (18) de type discoïdal.

6. Dispositif selon la revendication 5, **caractérisé par le fait que** le corps (18), de type discoïdal, présente une arête circulaire (32).

7. Dispositif selon la revendication 6, **caractérisé par le fait que** l'arête circulaire (32) s'étend sensiblement sur le pourtour d'une partie d'une circonférence.

8. Dispositif selon l'une des revendications 5 à 7, **caractérisé par le fait que** le corps (18) de type discoïdal comporte un orifice (44), en vue de la mise en place sur la tige creuse (12).

9. Dispositif selon la revendication 8, **caractérisé par le fait que** l'orifice (44) est non circulaire.

10. Dispositif selon la revendication 8, **caractérisé par le fait que** l'orifice (44) est circulaire.

11. Dispositif selon l'une des revendications 5 à 10, **caractérisé par le fait qu'**au moins un élément d'appui (24) est en débord au-delà du corps (18) de type discoïdal, vers le côté distal.

12. Dispositif selon la revendication 11, **caractérisé par le fait que** plusieurs éléments d'appui (24, 24'), du type picots, sont agencés avec débord.

13. Dispositif selon l'une des revendications 1 à 12, **caractérisé par le fait qu'**une poignée coudée (34) est implantée dans la région extrême proximale (42) de la tige creuse (12).
